(19)

Europäisches
Patentamt
European
Patent Office
Office européen
des brevets

(11) **EP 4 278 953 A1**

(12) **EUROPEAN PATENT APPLICATION**

(43) Date of publication:
**22.11.2023 Bulletin 2023/47**

(21) Application number: **22174355.2**

(22) Date of filing: **19.05.2022**

(51) International Patent Classification (IPC):
***A61B 5/00*** *(2006.01)*      ***G16H 50/20*** *(2018.01)*
***A61B 5/107*** *(2006.01)*      *A61B 5/103* *(2006.01)*

(52) Cooperative Patent Classification (CPC):
**A61B 5/0088; A61B 5/0077; A61B 5/1072;**
**A61B 5/4552; A61B 5/6898; A61B 5/7267;**
**A61B 5/7275; A61B 5/7282; G16H 30/40;**
**G16H 50/20; G16H 50/70;** A61B 5/1032;
A61B 5/4547; A61B 5/7405; A61B 5/742;      (Cont.)

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **KOOIJMAN, Gerben**
  **Eindhoven (NL)**
• **GERHARDT, Lutz Christian**
  **Eindhoven (NL)**
• **VAN LEEUWEN, Marinus Bastiaan**
  **5656AG Eindhoven (NL)**

(74) Representative: **Philips Intellectual Property &**
**Standards**
**High Tech Campus 52**
**5656 AG Eindhoven (NL)**

(54) **ASSESSING GUM RECESSION**

(57)      Proposed concepts aim to provide schemes, solutions, concepts, designs, methods and systems pertaining to the assessment of gum recession in a subject. In particular, embodiments of the invention gum line parameter values obtained from intraoral image(s). Specifically, a machine learning algorithm configured to process gum line parameter values describing a profile of the gums of the subject, in order to produce a gum recession score. The gum recession score may then be used to provide advice on brushing behaviour, or for the need for a profession check up, for example.

100

Obtain an intraoral image — 110

Generate gum line parameter values — 120

Provide the gum line parameter values to a machine learning algorithm — 130

Obtain a gum recession score from the machine learning algorithm — 140

FIG. 1

EP 4 278 953 A1

(52) Cooperative Patent Classification (CPC): (Cont.)
A61B 5/7465; A61B 2505/07

**Description**

FIELD OF THE INVENTION

The present invention relates to the field of gum recession assessment in subjects.

BACKGROUND OF THE INVENTION

The occurrence of receding gums or exposed roots is often associated with poor oral hygiene, calculus, bruxism, periodontitis, hormone-changes and incorrect brushing technique (e.g. excessive application of force). Receded gums can lead to dentine hypersensitivity, pain, infection, tooth decay and ultimately loss of teeth.

Gum recession often develops gradually within a timescale of months and years. However, it can also occur acutely in shorter time windows for reasons such as stress attacks, over-brushing, seasonal allergies, or injury caused by mechanical interdental cleaning. Typically, it is difficult to notice worsening of gum conditions by visual inspection, and thus such conditions are only noticed when symptoms such as pain or hypersensitivity arise.

Timely diagnosis is important as early stage treatment (e.g. pocket cleaning or antibiotics) can prevent or reverse gum recession, and thus prevent related gum diseases. A dental professional may examine a subject for gum recession by probing the pockets between teeth and gums. However, this process is time consuming and so is not usually performed in regular check-ups.

Therefore, for above-mentioned reasons, there exists a need for an objective tool to enable gum recession risk profiling for subjects.

SUMMARY OF THE INVENTION

The invention is defined by the claims.

According to examples in accordance with an aspect of the invention, there is provided a method for assessing gum recession in a subject, the method comprising:

obtaining an intraoral image of at least part of a subject's teeth and gums;

generating gum line parameter values describing a profile of the gums of the subject by analyzing the intraoral image;

providing the gum line parameter values to a machine learning algorithm, the machine learning algorithm being trained to calculate a presence of current gum recession in the subject or a likelihood that the subject is at risk of developing gum recession based on gum line parameter values; and

obtaining a gum recession score from the machine learning algorithm, the gum recession score indicative of a presence of gum recession in the subject or a risk of gum recession developing in the subject.

Proposed concepts thus aim to provide schemes, solutions, concepts, designs, methods and systems pertaining to the assessment of a risk of gum recession in a subject. In particular, embodiments of the invention gum line parameter values obtained from intraoral image(s). Specifically, a machine learning algorithm configured to process gum line parameter values describing a profile of the gums of the subject, in order to produce a gum recession score. The gum recession score may then be used to provide advice on brushing behaviour, or for the need for a profession check up, for example.

A proposed concept recognizes that gum line parameter values of gums of the subject may be leveraged in order to determine a risk of gum recession in the subject. Indeed, an analysis of a profile of gums of the subject may indicate a presence or potential presence in the future of the recession of the gums of the subject. It is also proposed that the gum line parameter values can be derived from an intraoral image (i.e. an image of the mouth) of the subject including teeth and gums. The intraoral image may be taken within the moth, or may be taken externally using a smartphone camera.

It has been realized that gum line parameter values describing the profile of the gum line of the gums of the user (i.e. in relation to the top of the crown of the users teeth, or the mucogingival junction) provide information that may be used to accurately calculate (i.e. predict) the likelihood that the subject suffers from or is likely to suffer from receding gums.

Embodiments may therefore be used to provide a gum recession score indicative of the probability/likelihood that the subject has receding gums, or is likely to have receded gums in the future. In this way, feedback may be provided in order for the subject to take proactive action to reduce/mitigate the impact of the receding gums, or to prevent the receding gums. For example, feedback regarding brushing behaviour or an oral care routine may be provided. Furthermore, the subject may be encouraged to have their gums checked by a dental professional. Alternatively, or additionally, a direct alert may be communicated to an oral care provider, which may be used to arrange a check-up. In this way, early detection and/or treatment of receding gums may be achieved, which may help to mitigate or even prevent the impact of the condition.

In other words, embodiments propose to use of machine learning to calculate the risk that the subject has, or may develop, gum recession. The machine learning algorithm/model may be more accurate, and/or may be able to interpret more from gum line parameter values than an observer can deduce from an intraoral image.

The machine learning algorithm may be trained using a training algorithm configured to receive an array of training inputs and known outputs, wherein the training inputs comprise gum line parameter values of a plurality of subjects and the known outputs comprise gum recession scores.

Thus, by exposing the machine learning algorithm to a plurality of different gum line parameter values with (known) gum recession scores, the algorithm may be able to provide an accurate (i.e. reflective of the ground truth) gum recession score for unseen gum line parameter values.

In some embodiments, the method may further comprise steps of comparing the gum line parameter values and historical gum line parameter values describing a previous profile of gums of the subject; and adjusting the gum recession score based on the comparison.

In this way, a more accurate gum recession score may be obtained. Indeed, gum recession is a condition that changes and presents over time. Therefore, a change in the gum line parameters with time may be indicative of the risk or presence of gum recession. This may be particularly true in instances where the gum recession is due to increased brushing force/mechanical trauma.

In other words, the machine learning algorithm may be particularly well adapted to determine a gum recession score from an instance (i.e. from one time) of gum parameter data. However, comparison with historical gum parameter values may be particularly useful to track a progression of gum recession, or lack thereof. So, by modifying the gum recession score using this method, a refined and accurate score may be obtained.

In some embodiments, the gum line parameter values may comprise gum line-mucogingival junction distance data describing a distance profile between a gum line of the subject and a mucogingival junction of the subject.

A particularly useful parameter relating to the profile of the gums is a distance between the gum line and the mucogingival junction. Put more simply, this is the distance between the top of the gums, and the start of the alveolar mucosa. Indeed, if the distance is relatively small then there may be a high chance of gum recession, and vice versa.

In additional embodiments, the gum line parameter values may comprise free tooth length data describing a distance profile between a gum line of the subject and a top part of a crown of a tooth of the subject.

Yet another useful parameter is the distance between where the gum line ends, and where the top of the tooth exists. In layman's terms, this indicates the amount of exposed tooth, and is therefore reflective of potential gum recession.

In further embodiments, the gum line parameter values may comprise profiliometric topography data describing oral surfaces of the subject. The profile (i.e. a roughness) of the oral surfaces may provide an indication not only of the type of oral surface (i.e. crown of the tooth, gums and alveolar mucosa), but also the thickness of such surfaces. Accordingly, this may indicate when such surfaces are degrading, and so may be a better reflection on the profile of the gum line. Thus, by utilizing this parameter value, a more accurate gum recession score may be generated.

In some embodiments, generating the gum line parameters may comprise processing, with an image analysis algorithm, the intraoral image in order to generate gum profile data describing a profile of the gums of the subject; and determining the gum line parameters based on the gum profile data.

An image analysis algorithm (e.g. a deep learning algorithm, or use of standard image processing tools that leverage color characteristics) may be utilized to ensure accurate gum line parameter values, and in turn a more accurate gum recession score. Indeed, the quality of the output of the machine learning algorithm is heavily dependent on the accuracy of the gum line parameter values. It may also be necessary to align images, and segment images for regions of interest in order to provide accurate gum line parameter values.

The method may further comprise steps of obtaining physiological data describing at least one physiological characteristic of the subject; and providing the physiological data to the machine learning algorithm, wherein the machine learning algorithm is trained to calculate a presence of current gum recession in the subject or a likelihood that the subject is at risk of developing gum recession based on gum line parameter values and physiological data.

Accordingly, the accuracy of the gum recession score may be improved. Indeed, different demographics may have different standard gum line profiles, and therefore it may be beneficial to analyze the gum line parameters differently depending on the demographic. For example, an older subject with non-receding gums may have a larger free tooth length than a younger subject with non-receding gums. In this case, the physiological data may include an age, a gender, an ethnicity, a smoking history, a diet history, an exercise history, a dental history, and a medical history of the subject.

In some embodiments, the method may further comprise generating oral care feedback based on the generated gum recession score; and communicating the oral care feedback to the subject, dental professional or a chatbot remotely connected to a recommendation platform or dentist. In this case, the oral care feedback may include at least one of oral care routine advice, and dental professional examination advice. Thus, embodiments may explicitly provide benefits to the subject by providing feedback/advice in relation to the gum recession score.

In some embodiments, the method may further comprise obtaining oral care device usage data describing usage of at least one oral care device on a subject. In which case, generating the oral care feedback may be further based on the oral care device usage data. The oral care device usage data may include at least one of device usage frequency data, device usage duration data, tooth coverage data, applied pressure data, scrubbing data, and oral care device type data. Oral care device usage data (i.e. toothbrush data, flossing data, or any data related to any treatment that may have an impact on gum properties), or data describing the brushing/cleaning habits of the user, may be utilized to better inform the user regarding action to be taken in response to the generated gum recession score. For example, if the score indicates that the subject's gums are starting to recede, it may be sufficient to advise brushing with less force, rather than a full check-up and treatment process.

According to further aspects of the invention, there is provided a computer program comprising computer program code means adapted, when said computer program is run on a computer, to implement a method for assessing gum recession in a subject.

According to yet another aspect of the invention, there is provided a system for assessing gum recession in a subject, the system comprising:

an image acquisition unit configured to obtain an intraoral image of at least part of a subject's teeth and gums;
an image analysis unit configured to generate gum line parameter values describing a profile of the gums of the subject by analyzing the image; and
an analysis unit configured to:

provide the gum line parameter values to a machine learning algorithm, the machine learning algorithm being trained to calculate a presence of current gum recession in the subject or a likelihood that the subject is at risk of developing gum recession based on gum line parameter values; and
obtain a gum recession score from the machine learning algorithm, the gum recession score indicative of a presence of gum recession in the subject or a risk of gum recession developing in the subject.

These and other aspects of the invention will be apparent from and elucidated with reference to the embodiments described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 is a flow diagram of a method for assessing gum recession in a subject according to an embodiment;
Figure 2 is a flow diagram of a method for adjusting the gum recession score generated according to the method of Figure 1;
Figure 3 is a flow diagram depicting a use of the method of Figure 1 and/or Figure 2;
Figure 4 presents an intra oral image, including example gum line parameters;
Figure 5 depicts the calculation of a gum line parameter value;
Figure 6 depicts an example of changing gum line parameter values with time;
Figure 7 depicts a simplified block diagram of a system for assessing gum recession in a subject according to an embodiment; and
Figure 8 is a simplified block diagram of a computer within which one or more parts of an embodiment may be employed.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

The invention proposes concepts for assessing gum recession in a subject. In this way, a subject may be warned pre-emptively regarding the status of their gum line. Indeed, it is often the case that receding gums are not noticed until pain and hypersensitivity have become an issue. Thus, the invention provides a tool that may help to reduce or prevent gum recession when still in an early stage.

Proposed concepts are based on the realisation that gum line parameter values may be derived from intraoral images. Such gum line parameter values are input to a trained machine learning algorithm in order to generate a gum recession score. Thus, proposed concepts may facilitate improved (i.e. more accurate) assessment of the presence of, or development of, gum recession in a subject.

Put another way, parameter values describing a gum profile extracted from an intraoral image of teeth and gums of the subject are leveraged to produce a gum recession (risk) score. The gum recession score represents the likelihood/chance that the subject has, or is likely to have, receding gums. More specifically, this is achieved by a machine learning algorithm configured to receive the gum line parameter values, and subsequently output a gum recession score associated with the subject.

According to proposed embodiments, the invention includes the following features:

(i) A device for obtaining an intraoral image. For example, a smartphone camera, tablet camera, digital photography camera, intraoral scanner, or oral care device with built-in camera may be utilised to obtain the image. In other embodiments, the intraoral image may be obtained from a dental database.

(ii) A first processing unit for analysing the intraoral image. Specifically, the processing unit may identify the gum line profile and/or profile of the mucogingival junction in an intra oral image, and derive certain gum line parameters/metrics.

The processing unit may also provide alignment such that the gum line profile may be comparable between images. The alignment may be, for instance, with the teeth index. This enables registration with previously captured image data from the same user, and/or extraction of a certain region of interest for gum line parameter value calculation when comparing to a database of images.

(iii) A memory unit to store the derived gum line parameter values, and optionally may store corresponding metadata (e.g. a time stamp describing when the intraoral image was taken). Additionally, the (relevant) part of the intraoral image may be stored.

(iv) A second processing unit (or second functionality of the first processing unit) for deriving a score indicative of a presence of gum recession in the subject or a likelihood of gum recession. This is based on a model taking as input the derived gum line parameters (and optionally physiological data of the subject such as age, gender, and dental record data). The model is a machine learning model/algorithm trained on a dataset containing derived gum line parameter values and corresponding/annotated scores, as well as above mentioned optional metadata for a plurality of subjects.

Furthermore, the derived gum recession score may be further based on a comparison of the derived gum line parameters with historic gum line parameters stored in the memory unit. In this way, changes to the subject's gum line profile over time may be taken into consideration.

(v) An interface for collecting and storing oral care (e.g. brushing data/device usage data, or any data related to the cleaning or treatment or oral tissue) of the user. The brushing data may, for example, comprise localization/coverage data (e.g. time-resolved pressure data per location, scrubbing data, etc.).

(vi) A feedback unit which, based on the derived gum recession (risk) score and (optional) brushing data, provides a feedback or recommendations to the subject with respect to brushing behaviour. In addition, or alternatively, the feedback unit may recommend a visit to a dental professional for further examination. As yet another alternative, the feedback unit may inform the dental professional regarding the subject's score, potentially planning a visit for examination automatically.

Indeed, many of the above features (processing, memory and feedback units) may be implemented in (part of) a smartphone, or a combination of (part of) a smartphone and a cloud service.

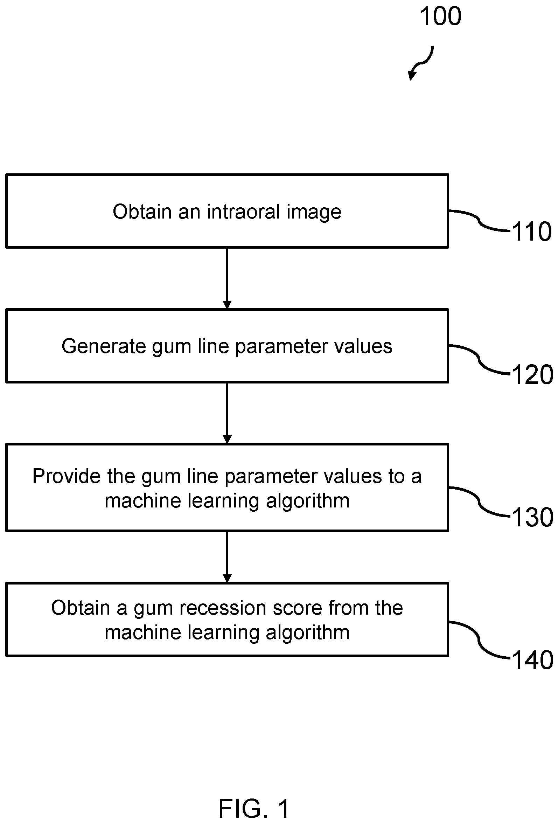

Figure 1 illustrates an embodiment of a method 100 (that may be computer-implemented) for assessing gum recession in a subject.

The method 100 begins with step 110 of obtaining an intraoral image of at least part of a subject's teeth and gums. The intraoral image (i.e. an image taken of the subject's mouth) must contain at least part of the subject's gum and teeth in order to be suitable for extracting gum line parameter values.

The intraoral image may be captured by a personal camera of the subject, or may be obtained by a dedicated mouthpiece. When the image is captured using a mouthpiece, the image may be well aligned such that extracted gum line parameter values are more accurate. However, obtaining the photo/image via a smartphone camera of the user may also be adequate.

In some embodiments, a subject may use their smartphone to capture the intraoral image. For this purpose, an application may be provided to instruct the subject, and give feedback regarding image quality. Such an application may also be used to give reminders to the subject, such that intraoral images are obtained on a regular basis.

Alternatively, for the purpose of this method 100, the image may be retrieved from a database or other storage means.

The method 100 then proceeds to step 120 of generating gum line parameter values. Specifically, the step 120 of generating gum line parameter values is achieved by analyzing the intraoral image.

The gum line parameter values describe a profile of the gums of the subject. Put another way, the gum line parameter values describe the shape/form of the gums of the subject. Indeed the gum line parameter values may describe the gum line of the subject relative to other features within the mouth of the subject.

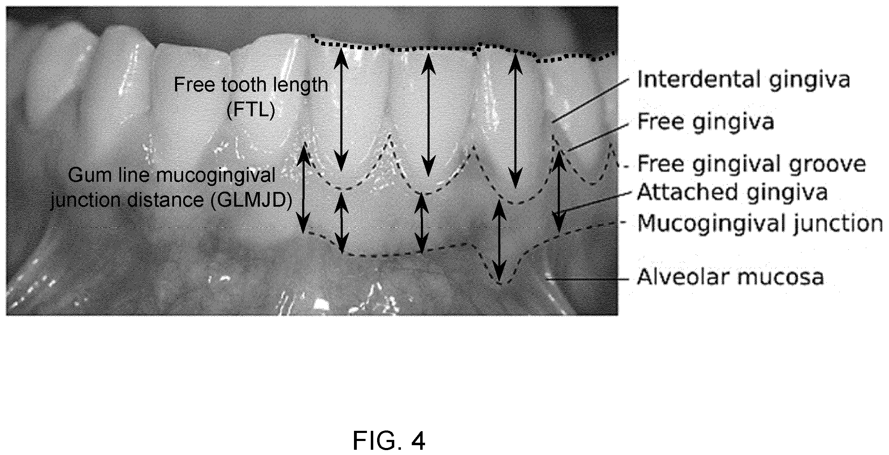

As shown in Figure 4, the gum line parameter values may comprise at least one of gum line-mucogingival junction distance (GLMJD) data, free tooth length (FTL) data, and topography data.

The GLMJD data describes a distance profile between a gum line of the subject and a mucogingival junction of the subject. In other words, this may describe a minimum distance between the mucogingival junction of the subject and the gum line, for each point of the gum line. By way of simplified example, when the distance/average distance is small, then this may indicate that the gums have receded.

The FTL data describes a distance profile between a gum line of the subject and a top part of a crown of a tooth of the subject. Put another way, the FTL data describes a minimum distance between the end protrusion of the teeth of the subject and the gum line, for each point of the gum line. Indeed, when the distance is large then this may indicate gum recession.

The profiliometric topography data describes a surface profile metric of two adjacent oral surfaces of the user. In other words, surface profile metrics equivalent to roughness or bearing area ratios from Abbott-Firestone curves may be used to identify changes in gum profile/gum recession.

Step 120 may also comprise processing, with an image analysis algorithm, the intraoral image in order to generate gum profile data describing a profile of the gums of the subject. This may be achieved with known image analysis tools, such as identifying stark colour differences, as is typically the case between gums and teeth. Alternatively, the image analysis algorithm may be a machine learning algorithm configured to analyse the image to determine a gum profile. The gum line parameters may then be derived from the gum profile data (i.e. by establishing distances between the gum line and other features of the mouth).

The gum line parameters, and derivation thereof, will be described in further detail in relation to Figures 4-7 below.

At step 130, the gum line parameter values are provided to a neural network (NN)-based machine learning algorithm (e.g. a deep learning NN). The machine learning algorithm is trained to calculate a presence of current gum recession in the subject or a likelihood that the subject is at risk of developing gum recession based on gum line parameter value. In certain embodiments, the machine learning algorithm is trained to predict the presence of current gum recession, or the chance that um recession will develop in the subject in the future.

The structure of an artificial NN (or, simply, neural network (NN)) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate calculated/predicted output data entries. An error between the predicted output data entries and corresponding training output data entries is used to modify the machine-learning algorithm. This process can be repeated until the error converges, and the predicted output data entries are sufficiently similar (e.g. $\pm 1\%$) to the training output data entries. This is commonly known as a supervised learning technique.

For example, weightings of the mathematical operation of each neuron may be modified until the error converges. Known methods of modifying a neural network include gradient descent, backpropagation algorithms and so on.

The training input data entries for the machine learning algorithm that may be used in method 100 correspond to gum line parameter values of a plurality of subjects and the known outputs comprise gum recession scores. The training output data entries correspond to gum recession scores, for each of the plurality gum line parameter values, indicating a presence of gum recession in the subject or a likelihood that the subject will develop gum recession in the future. That is, the machine learning algorithm is trained using a training algorithm configured to receive an array of training inputs and respective known outputs, wherein a training input comprises gum line parameter values of a plurality of subjects and respective known output comprises corresponding ground-truth gum recession scores. In this way, the machine learning algorithm is trained to calculate a presence of gum recession in the subject or a likelihood that the subject is at risk of developing gum recession based on gum line parameter value.

Furthermore, the method 100 may also comprise a step of obtaining physiological data describing at least one physiological characteristic of the subject. Indeed, this subject-specific data may provide contextual information that can be used to generate a more accurate gum recession score.

By way of example, the physiological data may include an age, a gender, an ethnicity, a smoking history, a diet history, an exercise history, a dental history, and a medical history of the subject. That is, the physiological data may include any variables that may affect the gum line profile.

In the case that the physiological data is obtained, the physiological data is provided to the machine learning algorithm. In this case, the machine learning algorithm is trained to calculate a presence of current gum recession in the subject or a likelihood that the subject is at risk of developing gum recession based on gum line parameter values and physiological data. Thus, the training inputs may comprise both gum line parameter values of a subject, and physiological data specific to the subjects.

At step 140, a result is obtained from the machine learning algorithm. The result comprises a gum recession score associated with the subject, which is either calculated or predicted. The gum recession score is indicative of a presence of gum recession in the subject or a risk of gum recession developing in the subject. In other words, the gum recession score represents a probability that the subject has receding gums, or a probability that the subject may have receding gums in the future.

By way of example, a low score may mean that the subject's gum line is not receding, and is unlikely to recede in the (near) future. A moderate score may mean that the subjects gums are at risk of receding, and therefore preventative measures should be taken to reduce the likelihood of gum recession. A high score may mean that the subject's gum line has receded, and thus may have exposed the roots of the teeth, and therefore treatment is required.

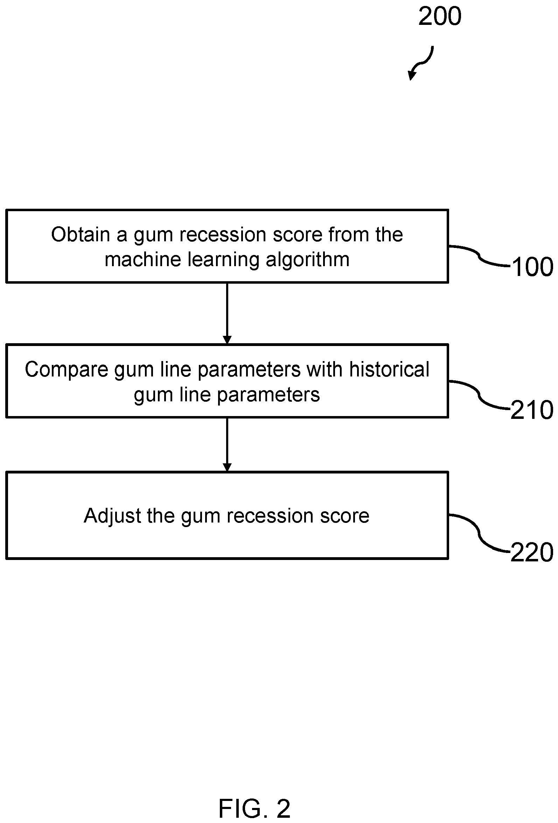

Figure 2 depicts a method 200 for improving/refining the gum recession score as obtained by the method 100 above. Indeed, the method 200 begins with implementing the method 100 of Figure 1 as described above, from which a gum recession score is generated.

Next, the method 200 proceeds to step 210 in which the (current) gum line parameter values of the subject are compared to historical gum line parameter values of the subject. In other words, the gum line parameters generated in step 120 of the method 100 of Figure 1 (and used to generate the gum recession score) are compared to previously generated gum line parameter values of the subject. Alternatively, gum line parameter data of other subjects with similar characteristics (e.g. from classified EMR data sets) may be compared to gum line parameter values of the subject.

Indeed, it is clear that gum recession is a condition that develops over time. Therefore, by assessing the change of gum line parameter values with time, clues regarding the presence of gum recession may be obtained. Thus, the gum recession score may be adjusted.

Put another way, if the historical gum line parameter values differ from the generated gum line parameter values above a threshold amount, this may indicate the presence of gum recession. In this case, the gum recession score may be adjusted accordingly.

Thus, at step 220, the gum recession score is adjusted/modified based on the comparison.

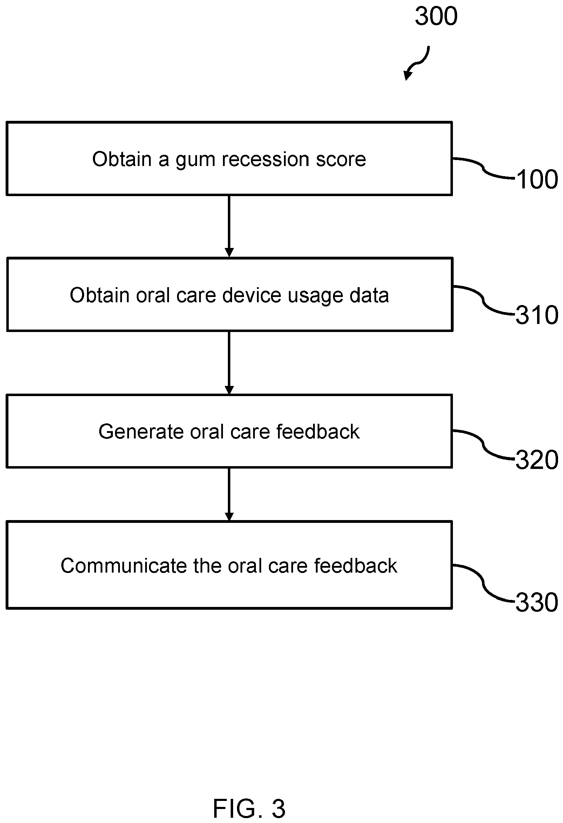

Figure 3 illustrates a use of the gum recession score obtained by either the method 100 of Figure 1, or modified by the method 200 of Figure 2. Indeed, in this instance the method 300 begins with implementing the method 100 of Figure 1 as described above, from which a gum recession score is obtained.

At step 310 (which is optional), oral care device usage data is obtained. This data describes the subject's usage of at least one oral care device. For instance, the device usage data may describe the subject's use of a toothbrush, a combined brushing flossing device, an irrigator, a mouthpiece, or a treatment device supplying electric, electromagnetic or acoustic energy. In this way, the subject's brushing routine/habits/behavior may be determined.

The oral care device usage data may include at least one of device usage frequency data, device usage duration data, tooth coverage data, applied pressure data, scrubbing data, and oral care device type data. In this way, both the time spent brushing, as well as the quality of brushing may be determined.

Moreover, the oral care device usage data may be obtained directly from a connected device (i.e. a smart toothbrush), or may be entered manually by the subject and/or a dental professional.

Next, in step 320, oral care feedback is generated based on the generated gum recession score. The oral care feedback may include at least one of oral care routine advice, and dental professional examination advice.

Feedback may be given regarding the subject's brushing behavior if, for example, the gum recession score indicates that the subject has receding gums. The advice may recommend that the subject uses less force when brushing, or brushes certain areas for a shorter amount of time.

Alternatively, if the gum recession score indicates severe recession of the gums, it may be recommended that the subject seeks examination by a dental professional.

In addition to the derived gum recession score, if the oral care device usage data has been obtained, the generated oral care feedback may dependent on the oral care device usage data.

In some embodiments, the following may be implemented:

(i) If the gum recession score does not exceed a predetermined threshold, no feedback/action may be taken, and thus no oral care feedback is provided.

(ii) If the gum recession score exceeds a first predetermined threshold (i.e. a medium risk threshold), then the feedback is provided to the user. When it is determined, from the oral care device usage data, that brushing pressure is high, a recommendation to brush with lower pressure may be given.

(iii) If the gum recession score exceeds a second, higher threshold (i.e. a high risk threshold), then an appointment for examination by a dentist is recommended or automatically scheduled. This may be performed regardless of the brushing information, as preventative measures may no longer be effective in isolation. The oral care device usage data may also be sent to the dental professional as part of the appointment preparation however.

Finally, in step 330, the oral care feedback may be communicated to the subject. This may be by visual or acoustic means. Indeed, an application may facilitate the communication. In some embodiments, an interactive avatar or chat-bot can immediately contact a dental professional and/or the subject to define follow-up actions based on the gum recession score.

Moving onto Figure 4, there is presented an example intraoral image that has been marked up to indicate example gum line parameters.

As shown, the free tooth length (FTL) is the length of tooth that is not covered by gum. The gum line mucogingival junction distance (GLMJD) is the amount of space between the free gingival groove and the mucogingival junction.

According to exemplary embodiments, from an intraoral image an image analysis algorithm detects the gum line profile, from which the gum line parameters may be derived. Traditional image analysis based techniques may be utilised, which typically utilise colour characteristics obtained from red and blue channels of the image.

Alternative, deep learning based algorithms may be used. For example, a deep learning based algorithm trained for segmentation of teeth versus non-teeth may be used. Alternatively, the deep learning algorithm may be trained to directly detect the tooth-gum transition. As another alternative, the deep learning algorithm may be trained to distinguish between detailed classes of objects, such as between front teeth, canine, molars, gum, exposed roots, etc.

For example, the gum line (which is roughly indicated as the free gingival groove in Figure 4), may be characterized by a ratio r that takes into account the average of the horizontal distances, $w_n$ between the free gingiva, and the average of the height fluctuations $h_n$. This may be indicated by the following equation:

$$r = \frac{\sum_N h_n}{\sum_N w_n} \qquad [1]$$

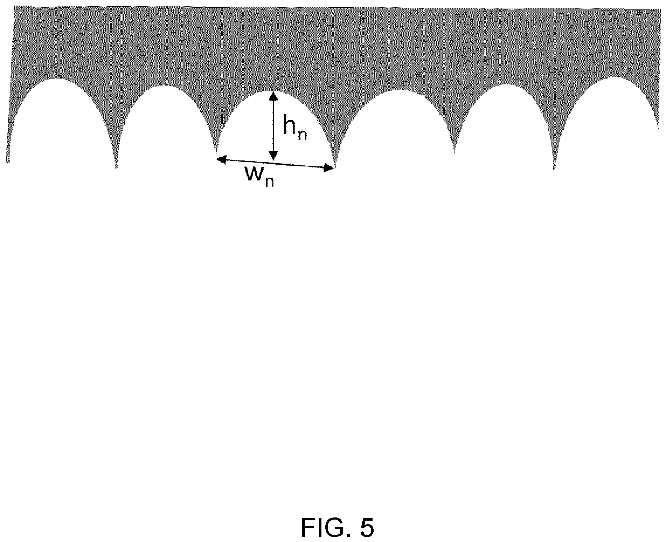

This is further illustrated in Figure 5, which shows a simplified representation of a gum line.

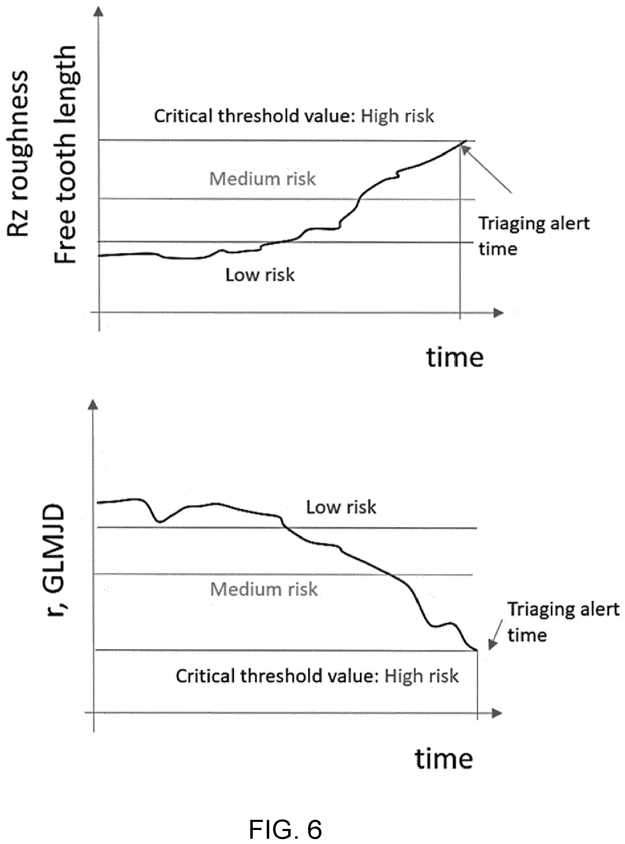

Figure 6 presents graphs depicting an example change over time of various gum line parameter values. By way of further explanation, some embodiments of the invention are based on the realisation that the development of gum line recession may be assessed by tracking a gum line parameter value over time. For this purpose, the derived gum line parameter values from an intraoral image may be stored in a memory, possibly with metadata such as a timestamp. For instance, a decreasing value of r overtime may indicate a progressing gum recession in the subject. A score proportional to the decrease in ratio r (compared to an initial value) may be determined. Alternatively, an increase in free tooth line (FTL) or profiliometric topography (i.e. a surface profile metric roughness depth) Rz, or decrease in gum line-mucogingival distance (GLMJD) can be used as a score and for stratification (assigning a particular risk status to a subject).

For accurate tracking of a gum line parameter over time, it may be necessary that the gum line parameter value is calculated for the same region of interest each time. Therefore, in the image analysis algorithm, alignment (e.g. with teeth) may be implemented. This alignment can be based on additional user input (e.g. user indicating the front teeth in the image), an algorithm trained to identify for instance the centre of the front teeth, or an advanced deep learning algorithm may be used to distinguish between front teeth, canines, and molars, which can be used for alignment.

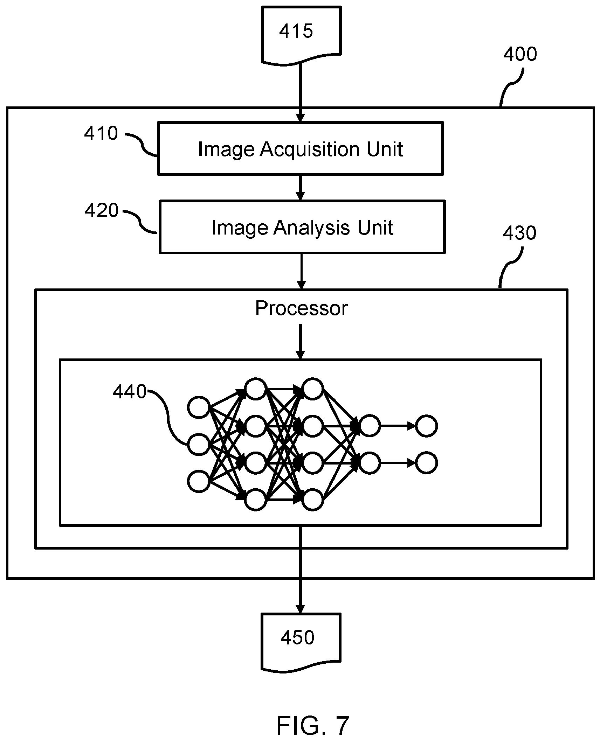

By the summary of a proposed implementation, Figure 7 depicts a simplified block diagram of a system 400 for assessing gum recession in a subject.

The system comprises an image acquisition unit 410 that is configured to obtain an intraoral image of at least part of a subject's teeth and gums. Further, the system 400 comprises an image analysis unit 420 configured to generate gum line parameter values describing a profile of the gums of the subject by analyzing the image. As outlined above, the gum line parameter values may include FTL data, GLMJD data, and topography data.

Furthermore, the system 400 comprises a processor 430, which is configured to provide the gum line parameter values to a machine learning algorithm 440. The machine learning algorithm 440 is configured to receive the gum line parameter values, and is trained to calculate a presence of current gum recession in the subject or a likelihood that the subject is at risk of developing gum recession based on the gum line parameter values.

The processor 430 is also configured to obtain a result from the machine learning algorithm 440. The (calculated/predicted) result is a gum recession score indicative of a presence of gum recession in the subject or a risk of gum recession developing in the subject. The gum recession score is then provided as output 440.

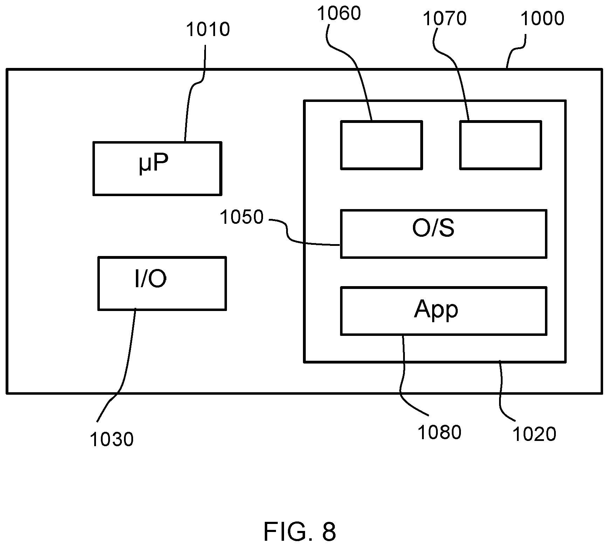

Figure 8 illustrates an example of a computer 1000 within which one or more parts of an embodiment may be employed. Various operations discussed above may utilize the capabilities of the computer 1000. For example, one or more parts of a system for asessing gum recession in a subject may be incorporated in any element, module, application, and/or component discussed herein. In this regard, it is to be understood that system functional blocks can run on a single computer or may be distributed over several computers and locations (e.g. connected via internet).

The computer 1000 includes, but is not limited to, PCs, workstations, laptops, PDAs, palm devices, servers, storages, and the like. Generally, in terms of hardware architecture, the computer 1000 may include one or more processors 1010, memory 1020 and one or more I/O devices 1030 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 1010 is a hardware device for executing software that can be stored in the memory 1020. The processor 1010 can be virtually any custom made or commercially available processor, a central processing unit (CPU), a digital signal processor (DSP), or an auxiliary processor among several processors associated with the computer 1000, and the processor 1010 may be a semiconductor based microprocessor (in the form of a microchip) or a microprocessor.

The memory 1020 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 1020 may incorporate electronic, magnetic, optical, and/or other types of storage media. Note that the memory 1020 can have a distributed architecture, where various components are situated remote from one another, but can be accessed by the processor 1010.

The software in the memory 1020 may include one or more separate programs, each of which comprises an ordered listing of executable instructions for implementing logical functions. The software in the memory 1020 includes a suitable operating system (O/S) 1050, compiler 1060, source code 1070, and one or more applications 1080 in accordance with exemplary embodiments. As illustrated, the application 1080 comprises numerous functional components for implementing the features and operations of the exemplary embodiments. The application 1080 of the computer 1000 may represent various applications, computational units, logic, functional units, processes, operations, virtual entities, and/or modules in accordance with exemplary embodiments, but the application 1080 is not meant to be a limitation.

The operating system 1050 controls the execution of other computer programs, and provides scheduling, input-output control, file and data management, memory management, and communication control and related services. It is contemplated by the inventors that the application 1080 for implementing exemplary embodiments may be applicable on all commercially available operating systems.

Application 1080 may be a source program, executable program (object code), script, or any other entity comprising a set of instructions to be performed. When a source program, then the program is usually translated via a compiler (such as the compiler 1060), assembler, interpreter, or the like, which may or may not be included within the memory 1020, so as to operate properly in connection with the O/S 1050. Furthermore, the application 1080 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 1030 may include input devices such as, for example but not limited to, a mouse, keyboard, scanner, microphone, camera, etc. Furthermore, the I/O devices 1030 may also include output devices, for example but not limited to a printer, display, etc. Finally, the I/O devices 1030 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 1030 also include components for communicating over various networks, such as the Internet or intranet.

If the computer 1000 is a PC, workstation, intelligent device or the like, the software in the memory 1020 may further include a basic input output system (BIOS) (omitted for simplicity). The BIOS is a set of essential software routines that initialize and test hardware at start-up, start the O/S 1050, and support the transfer of data among the hardware devices. The BIOS is stored in some type of read-only-memory, such as ROM, PROM, EPROM, EEPROM or the like, so that the BIOS can be executed when the computer 800 is activated.

When the computer 1000 is in operation, the processor 1010 is configured to execute software stored within the memory 1020, to communicate data to and from the memory 1020, and to generally control operations of the computer 1000 pursuant to the software. The application 1080 and the O/S 1050 are read, in whole or in part, by the processor 1010, perhaps buffered within the processor 1010, and then executed.

When the application 1080 is implemented in software it should be noted that the application 1080 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 1080 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figures 1-3 and the systems of Figure 7, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Figure 7 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

**Claims**

1. A method (100) for assessing gum recession in a subject, the method comprising:

    obtaining (110) an intraoral image of at least part of a subject's teeth and gums;
    generating (120) gum line parameter values describing a profile of the gums of the subject by analyzing the intraoral image;
    providing (130) the gum line parameter values to a machine learning algorithm, the machine learning algorithm being trained to calculate a presence of current gum recession in the subject or a likelihood that the subject is at risk of developing gum recession based on gum line parameter values; and
    obtaining (140) a gum recession score from the machine learning algorithm, the gum recession score indicative of a presence of gum recession in the subject or a risk of gum recession developing in the subject.

2. The method of claim 1, wherein the machine learning algorithm is trained using a training algorithm configured to receive an array of training inputs and known outputs, wherein the training inputs comprise gum line parameter values of a plurality of subjects and the known outputs comprise gum recession scores.

3. The method of claim 1 or 2, further comprising:

    comparing (210) the gum line parameter values and historical gum line parameter values describing a previous profile of gums of the subject; and
    adjusting (220) the gum recession score based on the comparison.

4. The method of any of claims 1-3, wherein the gum line parameter values comprise gum line-mucogingival junction distance data describing a distance profile between a gum line of the subject and a mucogingival junction of the subject.

5. The method of any of claims 1-4, wherein the gum line parameter values comprise free tooth length data describing a distance profile between a gum line of the subject and a top part of a crown of a tooth of the subject.

6. The method of any of claims 1-5, wherein the gum line parameter values comprise profiliometric topography data describing oral surfaces of the user.

7. The method of any of claims 1-6, wherein generating the gum line parameter values comprises:

    processing, with an image analysis algorithm, the intraoral image in order to generate gum profile data describing a profile of the gums of the subject; and
    determining the gum line parameters based on the gum profile data.

8. The method of any of claims 1-7, further comprising:

    obtaining physiological data describing at least one physiological characteristic of the subject; and
    providing the physiological data to the machine learning algorithm, wherein the machine learning algorithm is trained to predict a presence of gum recession in the subject or a likelihood that the subject is at risk of developing gum recession based on gum line parameter values and physiological data.

9. The method of claim 8, wherein the physiological data includes an age, a gender, an ethnicity, a smoking history, a diet history, an exercise history, a dental history, and a medical history of the subject.

10. The method of any of claims 1-9, further comprising:

    generating (320) oral care feedback based on the generated gum recession score; and
    communicating (330) the oral care feedback to the subject.

11. The method of claim 10, wherein the oral care feedback includes at least one of oral care routine advice, and dental professional examination advice.

12. The method of claim 10 or 11, further comprising obtaining (310) oral care device usage data describing usage of at least one oral care device on a subject, and wherein generating the oral care feedback is further based on the oral care device usage data.

13. The method of claim 12, wherein the oral care device usage data includes at least one of device usage frequency data, device usage duration data, tooth coverage data, applied pressure data, scrubbing data, and oral care device type data.

14. A computer program comprising computer program code means adapted, when said computer program is run on a computer, to implement the method of any of claims 1-13.

15. A system (400) for asessing gum recession in a subject, the system comprising:

an image acquisition unit (410) configured to obtain an intraoral image of at least part of a subject's teeth and gums;
an image analysis unit (420) configured to generate gum line parameter values describing a profile of the gums of the subject by analyzing the image; and
a processor (430) configured to:

provide the gum line parameter values to a machine learning algorithm (440), the machine learning algorithm being trained to calculate a presence of current gum recession in the subject or a likelihood that the subject is at risk of developing gum recession based on gum line parameter values; and
obtain a gum recession score from the machine learning algorithm, the gum recession score indicative of a presence of gum recession in the subject or a risk of gum recession developing in the subject.

100

```
┌─────────────────────────────────────────┐
│                                         │
│        Obtain an intraoral image        │──── 110
│                                         │
└─────────────────────────────────────────┘
                     │
                     ▼
┌─────────────────────────────────────────┐
│                                         │
│    Generate gum line parameter values   │──── 120
│                                         │
└─────────────────────────────────────────┘
                     │
                     ▼
┌─────────────────────────────────────────┐
│   Provide the gum line parameter        │
│   values to a                           │──── 130
│   machine learning algorithm            │
└─────────────────────────────────────────┘
                     │
                     ▼
┌─────────────────────────────────────────┐
│   Obtain a gum recession score from the │
│   machine learning algorithm            │──── 140
│                                         │
└─────────────────────────────────────────┘
```

FIG. 1

200

| Obtain a gum recession score from the machine learning algorithm | ~100 |
| Compare gum line parameters with historical gum line parameters | ~210 |
| Adjust the gum recession score | ~220 |

FIG. 2

300

```
┌─────────────────────────────────────────┐
│      Obtain a gum recession score        │──── 100
└─────────────────────────────────────────┘
                    │
                    ▼
┌─────────────────────────────────────────┐
│     Obtain oral care device usage data   │──── 310
└─────────────────────────────────────────┘
                    │
                    ▼
┌─────────────────────────────────────────┐
│       Generate oral care feedback        │──── 320
└─────────────────────────────────────────┘
                    │
                    ▼
┌─────────────────────────────────────────┐
│    Communicate the oral care feedback    │──── 330
└─────────────────────────────────────────┘
```

FIG. 3

FIG. 4

FIG. 5

FIG. 6

415

400

410 — Image Acquisition Unit

420 — Image Analysis Unit

430

Processor

440 — 

450

FIG. 7

FIG. 8

Europäisches
Patentamt

European
Patent Office

Office européen
des brevets

**PARTIAL EUROPEAN SEARCH REPORT**

under Rule 62a and/or 63 of the European Patent Convention.
This report shall be considered, for the purposes of
subsequent proceedings, as the European search report

**Application Number**

EP 22 17 4355

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2020 179173 A (SUNSTAR INC)<br>5 November 2020 (2020-11-05)<br>* figures 1-5 *<br>* paragraph [0013] *<br>* paragraph [0029] - paragraph [0070] * | 1,2,4-15 | INV.<br>A61B5/00<br>G16H50/20<br>A61B5/107 |
| X | WO 2022/085724 A1 (SUNSTAR INC [JP])<br>28 April 2022 (2022-04-28)<br>* figures 1-11 *<br>* paragraph [0036] - paragraph [0053] * | 1,2,4-15 | ADD.<br>A61B5/103 |
| X | WO 2021/064114 A1 (ADENT APS [DK])<br>8 April 2021 (2021-04-08)<br>* figures 1-5 *<br>* page 18, line 22 - page 27, line 29 * | 1,2,4-15 | |
| A | US 2018/168781 A1 (KOPELMAN AVI [US] ET AL) 21 June 2018 (2018-06-21)<br>* paragraph [0099] - paragraph [0100] * | 4-6 | |
| A | EP 3 553 518 A1 (KONINKLIJKE PHILIPS NV [NL]) 16 October 2019 (2019-10-16)<br>* paragraph [0074] *<br>-----<br>-/-- | 4-6 | **TECHNICAL FIELDS SEARCHED (IPC)**<br><br>A61B<br>G16H<br>A61D<br>A46B |

## INCOMPLETE SEARCH

The Search Division considers that the present application, or one or more of its claims, does/do
not comply with the EPC so that only a partial search (R.62a, 63) has been carried out.

Claims searched completely :

Claims searched incompletely :

Claims not searched :

Reason for the limitation of the search:

**see sheet C**

| Place of search | Date of completion of the search | Examiner |
|---|---|---|
| The Hague | 17 January 2023 | Knoop, Jan |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

**page 1 of 2**

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| A | MARKO KURALT ET AL: "The precision of gingival recession measurements is increased by an automated curvature analysis method", BMC ORAL HEALTH, BIOMED CENTRAL LTD, LONDON, UK, vol. 21, no. 1, 7 October 2021 (2021-10-07), pages 1-10, XP021297100, DOI: 10.1186/S12903-021-01858-9 * abstract; figure 1 * * the whole document * ----- | 6 | |
| E | EP 4 113 440 A1 (KONINKLIJKE PHILIPS NV [NL]) 4 January 2023 (2023-01-04) * figures 1-4 * * paragraph [0048] * ----- | 1,2,6, 14,15 | **TECHNICAL FIELDS SEARCHED     (IPC)** |

EPO FORM 1503 03.82 (P04C10)

**page 2 of 2**

Europäisches
Patentamt

European
Patent Office

Office européen
des brevets

**INCOMPLETE SEARCH**
**SHEET C**

Claim(s) searched incompletely:
        1, 2, 4-15


Claim(s) not searched:
        3


Reason for the limitation of the search:


1           Clarity and Conciseness (Art. 84 EPC)
The application does not meet the requirements of Article 84 EPC, because claims 1-15 are not clear.
1.2         Claim 1 is not clear, as it fails to define how the machine learning algorithm was trained. For arriving at such an algorithm, it has to be trained based on labelled data containing e. g. the gum recession scores on the basis of input parameters, i. e. the gum line parameters. Consequently, claim 2 should be integrated into claim 1. Similarly, claim 15 is also unclear.
1.3         Consequently, claim 1 and 15 are searched limited to the subject-matter contained in dependent claim 2.
1           Sufficiency of disclosure (Art. 83 EPC)
The scope of protection of dependent claim 3 is not sufficiently disclosed in the application as a whole in a way that the skilled person could carry it out. Therefore, an objection under Article 83 EPC is raised.
1.1.1       Specifically, it is unclear how an already obtained gum recession score on the basis of the output of the ML algorithm should be adjusted in view of previous a comparison of current and previous gum line parameters of a subject. Should a modification be performed when there is no (significant) change between measurements? Should the score be reduced, indicating less risk of gum recession? Or should it be increased, as no improvement in a condition has been met? How would the skilled person modify these scores in these circumstances when they are high (and thereby indicative of gumline recession) or low (and thereby not indicating gumline recession)? What would the skilled person do if there is a change in gumline parameters between a current and previous measurement? Would it depend on the passed amount of time between the measurements? As the application fails to address these questions, it is not clear to the skilled person which conclusions to draw from the comparison between current and previous gumline parameters, and how to adjust the score on said basis.

## ANNEX TO THE EUROPEAN SEARCH REPORT
## ON EUROPEAN PATENT APPLICATION NO.

EP 22 17 4355

This annex lists the patent family members relating to the patent documents cited in the above-mentioned European search report.
The members are as contained in the European Patent Office EDP file on
The European Patent Office is in no way liable for these particulars which are merely given for the purpose of information.

17-01-2023

| Patent document cited in search report | | | Publication date | Patent family member(s) | | | Publication date |
|---|---|---|---|---|---|---|---|
| JP 2020179173 | A | | 05-11-2020 | NONE | | | |
| WO 2022085724 | A1 | | 28-04-2022 | NONE | | | |
| WO 2021064114 | A1 | | 08-04-2021 | DK | 201970623 | A1 | 01-07-2021 |
| | | | | EP | 4022648 | A1 | 06-07-2022 |
| | | | | US | 2022351500 | A1 | 03-11-2022 |
| | | | | WO | 2021064114 | A1 | 08-04-2021 |
| US 2018168781 | A1 | | 21-06-2018 | US | 2018168780 | A1 | 21-06-2018 |
| | | | | US | 2018168781 | A1 | 21-06-2018 |
| | | | | US | 2020237486 | A1 | 30-07-2020 |
| | | | | US | 2021121271 | A1 | 29-04-2021 |
| | | | | US | 2021298878 | A1 | 30-09-2021 |
| | | | | WO | 2018112273 | A2 | 21-06-2018 |
| EP 3553518 | A1 | | 16-10-2019 | CN | 111954815 | A | 17-11-2020 |
| | | | | EP | 3553518 | A1 | 16-10-2019 |
| | | | | EP | 3775905 | A2 | 17-02-2021 |
| | | | | JP | 2021521425 | A | 26-08-2021 |
| | | | | US | 2021109113 | A1 | 15-04-2021 |
| | | | | WO | 2019197543 | A2 | 17-10-2019 |
| EP 4113440 | A1 | | 04-01-2023 | EP | 4113440 | A1 | 04-01-2023 |
| | | | | WO | 2023274690 | A1 | 05-01-2023 |

EPO FORM P0459

For more details about this annex : see Official Journal of the European Patent Office, No. 12/82